# EUROPEAN PATENT APPLICATION

(11) **EP 4 628 139 A1**
(43) Date of publication of application: **08.10.2025**
(21) Application number: 23896204.7
(22) Date of filing: 14.09.2023
(51) Int. Cl.: A61M 25/01, A61F 2/24

(54) **DELIVERY SYSTEM**

(30) Priority: 01.12.2022 CN 202211527659
(71) Applicant: Shanghai MicroPort CardioFlow Medtech Co., Ltd., Shanghai 201203 (CN)
(72) Inventor: YU, Wenxia, Shanghai 201203 (CN); ZHANG, Pinghai, Shanghai 201203 (CN); HUANG, Qingqing, Shanghai 201203 (CN); CHEN, Guoming, Shanghai 201203 (CN)
(74) Representative: Patentship Patentanwaltsgesellschaft mbH
(86) International application number: PCT/CN2023/118863
(87) International publication number: WO 2024/114047

(57) **Abstract**

The present application relates to a delivery system, an inner catheter (2000) is movably disposed in an axial lumen of an outer catheter (1000). The inner catheter (2000) includes a pushing inner catheter section (1100) and a bending inner catheter section (1200) along an axis. A diameter of the bending inner catheter section (1200) is greater diameter than a diameter of the pushing inner catheter section (1100). A guide wire (2100b) is inserted within a guide lumen of the inner catheter (2000), and a pull wire (2200b) is connected to the bending inner catheter section (1200).

## Description

### RELATED APPLICATION

This application claims priority to Chinese Patent Application No. 2022115276593 filed on December 01, 2022, entitled "Delivery System", which is hereby incorporated by reference in its entirety.

### TECHNICAL FIELD

The present application relates to the technical field of medical devices, and particularly to a delivery system.

### BACKGROUND

Transcatheter aortic valve replacement (TAVR) is a procedure to deliver, position and release a prosthetic aortic valve at the aortic root to replace the native valve using a catheter of a delivery system. TAVR is a popular technique in the field of valvular heart disease treatment, and a milestone in minimally invasive intervention of aortic valve disease. Treatment of aortic valve disease by TAVR does not require opening the chest or stopping the heart of a patient, which will otherwise cause huge trauma to the patient.

Catheters in most existing delivery systems for use in TAVR procedures are not actively steerable. In order to pass through the aortic arch, they have to bend passively under the action of reaction forces from the inner wall of the blood vessel. However, in this process, some damage may be caused to the blood vessel inner wall, possibly leading to some vascular complications. Further, due to the absence of active steerability of such catheters, annulus crossing and coaxial release in a later TAVR stage would require very difficult operations, affecting release and anchoring of the valve. Therefore, how to achieve active steerability of a catheter of a delivery system remains a challenging issue requiring urgent resolution.

### SUMMARY OF THE INVENTION

Delivery systems are proposed in various embodiments disclosed herein.

Herein, there is provided a delivery system comprising:
an outer catheter comprising an axial lumen;
an inner catheter comprising a guide lumen, wherein the inner catheter is movably disposed in the axial lumen of the outer catheter, wherein the inner catheter comprises an pushing inner catheter section and an bending inner catheter section along an axial direction, wherein a distal end of the pushing inner catheter section is joined to a proximal end of the bending inner catheter section, and wherein a diameter of the bending inner catheter section is greater than a diameter of the pushing inner catheter section;
a guide wire inserted in the guide lumen of the inner catheter; and
a pull wire connected to the bending inner catheter section.

In one embodiment, a distal end of the pull wire is connected to a distal end of the bending inner catheter section.

In one embodiment, a pulling fixing member is provided on the bending inner catheter section, and wherein the pull wire is connected to the bending inner catheter section through the pulling fixing member.

In one embodiment, the pulling fixing member comprises a first inner bore in communication with the guide lumen.

In one embodiment, the pulling fixing member is provided with a first wire attachment member, and the pull wire is connected to the first wire attachment member.

In one embodiment, the inner catheter comprises a pulling lumen, and the pull wire is inserted in the pulling lumen of the inner catheter.

In one embodiment, a bend-resistant support tube is provided in the pulling lumen.

In one embodiment, a proximal end of the pushing inner catheter section is provided with an inner-catheter coupling segment, wherein a wire exit opening is provided on the inner-catheter coupling segment, and wherein the wire exit opening is in communication with the pulling lumen, and wherein a proximal end of the pull wire is guided out through the wire exit opening.

In one embodiment, a proximal end of the bending inner catheter section has a diameter-varying segment that is joined to the distal end of the pushing inner catheter section, wherein a diameter of the diameter-varying segment of the bending inner catheter section gradually decreases from a distal end to a proximal end.

In one embodiment, the bending inner catheter section is provided with a stent pulling fixing member.

In one embodiment, the stent fixing member is provided at the proximal end of the bending inner catheter section, and wherein the bending inner catheter section is connected to the distal end of the pushing inner catheter section through the stent pulling fixing member.

In one embodiment, the stent fixing member is provided at a distal end of the bending inner catheter section.

In one embodiment, the bending inner catheter section comprises a first inner-catheter segment and a second inner-catheter segment along an axial direction, wherein a distal end of the first inner-catheter segment is coupled to a proximal end of the second inner-catheter segment by the stent pulling fixing member.

In one embodiment, the stent fixing member comprises a second inner bore in communication with the guide lumen.

In one embodiment, the stent fixing member has a stent fixing element.

In one embodiment, the bending inner catheter section comprises a bendable backbone and a bendable protective layer, the bendable backbone disposed within the bendable protective layer.

In one embodiment, the bendable backbone comprises a backbone tube, and a wall of the backbone tube is provided with a bend-assisting opening.

In one embodiment, the bend-assisting opening is a linear opening, wherein the bend-assisting opening is arranged circumferentially around the backbone tube, wherein a plurality of bend-assisting openings are provided, and wherein the plurality of bend-assisting openings are distributed along an axis of the backbone tube.

In one embodiment, the bendable protective layer comprises an inner bendable protective layer and an outer bendable protective layer, the outer bendable protective layer provided on an outer surface of the backbone tube, the inner bendable protective layer provided on an inner surface of the backbone tube.

In one embodiment, the bendable backbone comprises a plurality of rotatable elements that are distributed along an axis of the bendable backbone, wherein adjacent rotatable elements are articulated.

In one embodiment, adjacent rotatable elements are rotationally coupled with fixed-axis, and wherein a plurality of rotatable axes of the plurality of rotatable elements are parallel.

In one embodiment, the pushing inner catheter section comprises an inner-catheter inner layer and an inner-catheter outer layer, wherein an inner-catheter braided layer is disposed between the inner-catheter inner layer and the inner-catheter outer layer, and at least one of the guide lumen and the pulling lumen is formed within the inner-catheter inner layer.

In one embodiment, at least one inner-catheter reinforcement element is disposed within the inner-catheter inner layer and is located between the guide lumen and the pulling lumen.

In one embodiment, the outer catheter comprises a pushing outer catheter section and a bending outer catheter section along an axis direction, a distal end of the pushing outer catheter section joined to a proximal end of the bending outer catheter section.

In one embodiment, a distal end of the bending outer catheter section has a deformable segment having a straight cylindrical configuration and a tapered cylindrical configuration, wherein the deformable segment is configured to transition between the straight cylindrical configuration and the tapered cylindrical configuration.

In one embodiment, a proximal end of the bending outer catheter section has a diameter-varying segment joined to the distal end of the pushing outer catheter section, wherein a diameter of the diameter-varying segment of the bending outer catheter section gradually decreases from a distal end to a proximal end.

The details of one or more embodiments of this application are set forth in the accompanying drawings and the description below. Other features, objects, and advantages of the disclosure will be apparent from the description and drawings, and from the appended claims.

### BRIEF DESCRIPTION OF THE DRAWINGS

In order to more clearly explain the techniques proposed herein or of the prior art, the accompanying drawings, to which reference is to be made in connection with the following description of embodiments or the prior art, will be briefed below. Apparently, these drawings show only some embodiments of the application, and those of ordinary skill in the art can obtain other drawings in light of those contained herein, without paying any creative effort.
Fig. 1 schematically illustrates how an inner catheter is steered in accordance with some embodiments of the present application.
Fig. 2 is a schematic diagram showing the structure of an outer catheter according to some embodiments of the present application.
Fig. 3 is a schematic diagram showing the structure of a delivery system according to some embodiments of the present application.
Fig. 4 is a schematic diagram showing the structure of a bending outer catheter section according to some embodiments of the present application.
Fig. 5 is an enlarged partial view (I) of the bending outer catheter section of Fig. 4.
Fig. 6 is another enlarged partial view (II) of the bending outer catheter section of Fig. 4.
Fig. 7 is a schematic diagram showing the structure of a backbone tube according to some embodiments of the present application.
Fig. 8 is a schematic diagram showing the structure of a backbone tube according to some other embodiments of the present application.
Fig. 9 is a schematic cross-sectional view of a pushing inner catheter section according to some embodiments of the present application.
Fig. 10 is a schematic diagram showing the structure of an inner-catheter coupling segment according to some embodiments of the present application.
Fig. 11 is a schematic diagram showing the structure of an inner catheter according to some embodiments of the present application.
Fig. 12 is a schematic illustration of the inner catheter of the Fig. 11 being in a bent configuration.
Fig. 13 is a schematic diagram showing the structure of a pulling fixing member according to some embodiments of the present application.
Fig. 14 is a schematic diagram showing the structure of a stent fixing member according to some embodiments of the present application.
Fig. 15 is a schematic perspective view of an assembly of a pulling fixing member, a backbone tube and a stent fixing member according to some embodiments of the present application.
Fig. 16 is a schematic plan view of an assembly of a pulling fixing member, a backbone tube and a stent fixing member according to some embodiments of the present application.
Figs. 17 to 21 schematically illustrate a delivery system being in operation according to some embodiments of the present application.
Fig. 22 is a schematic diagram showing the structure of an inner catheter according to some other embodiments of the present application.
Fig. 23 is a schematic illustration of the inner catheter of the Fig. 22 being in a bent configuration.
Fig. 24 is a schematic perspective view of an assembly of a pulling fixing member, a backbone tube and a stent fixing member according to some other embodiments of the present application.
Fig. 25 is a schematic plan view of an assembly of a pulling fixing member, a backbone tube and a stent fixing member according to some other embodiments of the present application.
Figs. 26 to 29 schematically illustrate a delivery system being in operation according to some other embodiments of the present application.
Fig. 30 is a schematic diagram showing the structure of an inner catheter according to some further embodiments of the present application.
Fig. 31 is a schematic illustration of the inner catheter of the Fig. 30 being in a bent configuration.
Fig. 32 is a schematic diagram showing the structure of a stent fixing member according to some other embodiments of the present application.
Fig. 33 is a schematic perspective view of an assembly of a pulling fixing member, a backbone tube and a stent fixing member according to some further embodiments of the present application.
Fig. 34 is a schematic plan view of an assembly of a pulling fixing member, a backbone tube and a stent fixing member according to some further embodiments of the present application.
Figs. 35 to 38 schematically illustrate a delivery system being in operation according to some embodiments of the present application.

List of Reference Numerals
A, aortic arch; B, plane of valvular annulus; C, prosthetic valve; D, stent body; E, point of support; F, point of anchoring; G, direction of withdrawal;
1000, outer catheter; 2000, inner catheter; 3000, grip member;
1100, pushing outer catheter section; 1200, bending outer catheter section; 1300, coupling ring;
1110, outer-catheter reinforcement member; 1110a, welded position; 1120, outer-catheter coupling segment; 1210, reinforcement structure; 1220 deformable segment;
2100a, guide lumen; 2100b, guide wire; 2200a, pulling lumen; 2200b, pull wire; 2200c, bend-resistant support tube;
2100, pushing inner catheter section; 2200, bending inner catheter section; 2300, inner-catheter coupling segment;
2300a, first coupling segment; 2300b, second coupling segment;
2110 inner-catheter inner layer; 2120 inner-catheter outer layer; 2130, inner-catheter braided layer; 2140, inner-catheter reinforcement element;
2210, pulling fixing member; 2220, stent pulling fixing member; 2230, first inner-catheter segment; 2240, second inner-catheter segment; 2250, bendable backbone; 2260, fusion connection claw;
2310, wire exit opening;
2210a, first inner bore; 2210b, first wire attachment member; 2220a, second inner bore; 2220b, lateral pull-wire hole; 2220c, stent fixing element; 2220d, second wire attachment member;
2251, backbone tube; 2251a, bend-assisting opening; 2252, pivotable element;
3100, rotary control member; 3200, thread.

### DETAILED DESCRIPTION

Embodiments of the present application will be described clearly and fully hereunder in conjunction with the appended drawings. Evidently, the embodiments set forth herein are merely some but not all possible embodiments of the application. Any and all other embodiments devisable by skilled artisans in light of the disclosed embodiments without paying any creative effort are considered to fall within the scope of protection of this application.

In order to more clearly describe the structure of delivery system proposed in this application, the term "distal end" may be used herein to refer to an end away from an operator during a surgical procedure, and "proximal end" to an end closer to the operator during a surgical procedure. Unless defined otherwise, all technical and scientific terms used herein have the same meaning as commonly understood by one of ordinary skill in the art to which this application belongs. The terminology used herein is for the purpose of describing particular embodiments only and is not intended to be limiting of the application.

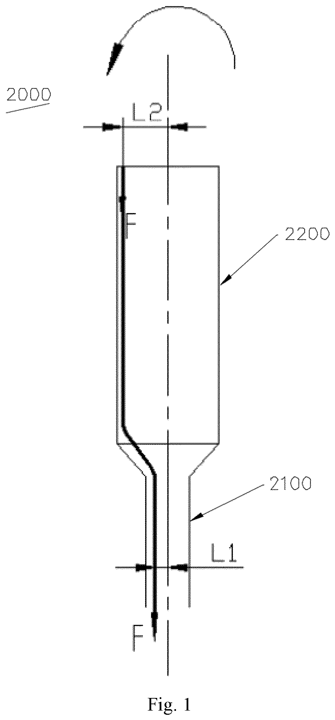

Referring to Figs. 1 to 3, in an embodiment of the present application, there is provided a delivery system including an outer catheter 1000, an inner catheter 2000, a guide wire 2100b and a pull wire 2200b. The outer catheter 1000 comprises an axial lumen, and the inner catheter 2000 comprises a guide lumen 2100a. The inner catheter 2000 is movably disposed in the axial lumen of the outer catheter 1000. The inner catheter 2000 includes a pushing inner catheter section 2100 and a bending inner catheter section 2200 along an axis, and a distal end of the pushing inner catheter section 2100 is joined to a proximal end of the bending inner catheter section 2200. A diameter of the bending inner catheter section 2200 is greater than a diameter of the pushing inner catheter section 2100. The guide wire 2100b is inserted in the guide lumen 2100a of the inner catheter 2000, and the pull wire 2200b is connected to the bending inner catheter section 2200.

By "the inner catheter 2000 is movably disposed in the axial lumen of the outer catheter 1000", it is essentially intended to mean that the inner catheter 2000 is able to axially move and circumferentially rotate within the axial lumen of the outer catheter 1000 relative to the outer catheter 1000. A stent body D with a valve C can be disposed between the inner catheter 2000 and the outer catheter 1000, or disposed on the bending inner catheter section 2200 of the inner catheter 2000. In one embodiment, the outer catheter 1000 may also include a pushing outer catheter section 1100 and a bending outer catheter section 1200 along an axis, and a distal end of the pushing outer catheter section 1100 may be joined to a proximal end of the bending outer catheter section 1200. In this case, the pushing outer catheter section 1100 of the outer catheter 1000 may correspond to and match the pushing inner catheter section 2100 of the inner catheter 2000 in structure, and the bending outer catheter section 1200 of the outer catheter 1000 may correspond to and match the bending inner catheter section 2200 of the inner catheter 2000 in structure. A length of the bending outer catheter section 1200 may be adjusted based on the bending control requirement, and the stent body D with the valve C can be disposed between the bending inner catheter section 2200 of the inner catheter 2000 and the bending outer catheter section 1200 of the outer catheter 1000.

With continued reference to Fig. 1, when a connection location of the pull wire 2200b and the bending inner catheter section 2200 is determined, retracting the pull wire 2200b will pull the bending inner catheter section 2200 at said location. That is, when a force F is applied, the pull wire 2200b causes the bending inner catheter section 2200 to bend. Since a diameter of the bending inner catheter section 2200 is greater than diameter of the pushing inner catheter section 2100, pulling the large-diameter bending inner catheter section 2200 via the pull wire 2200b can create a relatively large moment perpendicular to a cross-section of the bending inner catheter section 2200, which enables good bending control effect. In other words, increasing the outer diameter of the bending inner catheter section 2200 and reducing modulus of the bending inner catheter section 2200 allow the inner catheter 2000 to achieve a large bending angle.

In order to match the diameter-varying bending control of the inner catheter 2000, the bending outer tube section 1200 can be designed with extended length to offer a larger space, without limiting the present application. In addition, the pull wire 2200b may be connected to the bending inner catheter section 2200 at any location. For example, in one embodiment, a distal end of the pull wire 2200b may be connected to a distal end, a middle portion or another location of the bending inner catheter section 2200. Those skilled in the art can adjust the connection location of the pull wire 2200b and the bending inner catheter section 2200 based on the bending control requirement of the bending inner catheter section 2200, without limiting the present application.

The pull wire 2200b may be connected to the bending inner catheter section 2200, either directly or indirectly. For example, the pull wire 2200b may be connected to the bending inner catheter section 2200 by ligation, entwinement, welding or the like. Alternatively, the bending inner catheter section 2200 may be provided with a pulling fixing member 2210 for connecting the pull wire 2200b to the bending inner catheter section 2200. In this case, the location of the pull wire 2200b on the bending inner catheter section 2200 is the location of the pulling fixing member 2210 on the bending inner catheter section 2200.

While being connected to the bending inner catheter section 2200, the pull wire 2200b can extend along the axial direction to the proximal end, for example, the pull wire 2200b extends in the axial direction through the gap between the inner catheter 2000 and the outer catheter 1000 to the proximal end. Alternatively, the inner catheter 2000 may comprise a pulling lumen 2200a, and the pull wire 2200b may extend in the axial direction to the proximal end by inserting the pull wire 2200b in the pulling lumen 2200a of the inner catheter 2000. Since the pull wire 2200b is disposed within the inner catheter 2000, the delivery system can be distally bent by manipulating the pull wire 2200b while not affecting relative axial movability of the inner catheter 2000 and the outer catheter 1000, e.g. advancement and retraction of the outer catheter 1000 relative to the inner catheter 2000, advancement and retraction of the outer catheter 1000 controls release and retrieval of the stent body D with the valve C. Without limitation, the advancement and retraction of the outer catheter 1000 may be controlled by a button.

Since the diameter of the bending inner catheter section 2200 is greater than diameter of the pushing inner catheter section 2100, the joint location of the pushing inner catheter section 2100 and the bending inner catheter section 2200 may be a diameter-varying transition or a stepped transition. For example, the proximal end of the bending inner catheter section 2200 has a diameter-varying segment joined to the distal end of the pushing inner catheter section 2100. The diameter of the diameter-varying section of the bending inner tube 2200 progressively decreases from the distal end to the proximal end, thereby forming a transition connection between the bending inner tube 2200 and pushing inner tube 2100 with different diameters. Additionally, the pulling lumen 2200a extending through the bending inner catheter section 2200 and the pushing inner catheter section 2100 varies its radial distance from the central axis of the inner catheter 2000 in accordance with diameter changes of the bending inner catheter section 2200 and the pushing inner catheter section 2100. With continued reference to Fig. 1, along a pulling force F applied to the pull wire 2200b, the pulling lumen 2200a and the pull wire 2200b therein may have radial distances of L2 and L1, respectively, at the bending inner catheter section 2200 and the pushing inner catheter section 2100, where L2 is less than L1.

The bending outer catheter section 1200 may be may be composed of multiple layers of different materials. For example, the bending outer catheter section 1200 may have an outer polymer layer, an inner polymer layer and a metal cutting reinforcement layer located between the outer and inner polymer layers. The outer polymer layer may be made of a flexible material such as polyurethane or a polyamide polyether copolymer, and the inner polymer layer may be made of a flexible material such as polytetrafluoroethylene, polyurethane or a polyamide polyether copolymer. Since the bending outer catheter section 1200 is required to have easy bendability, high compression resistance, good hoop stiffness and other properties, the metal cutting reinforcement layer may be made of a NiTi alloy material with good elasticity. Those skilled in the art may make the bending outer catheter section 1200 based on needs, without limiting the present application.

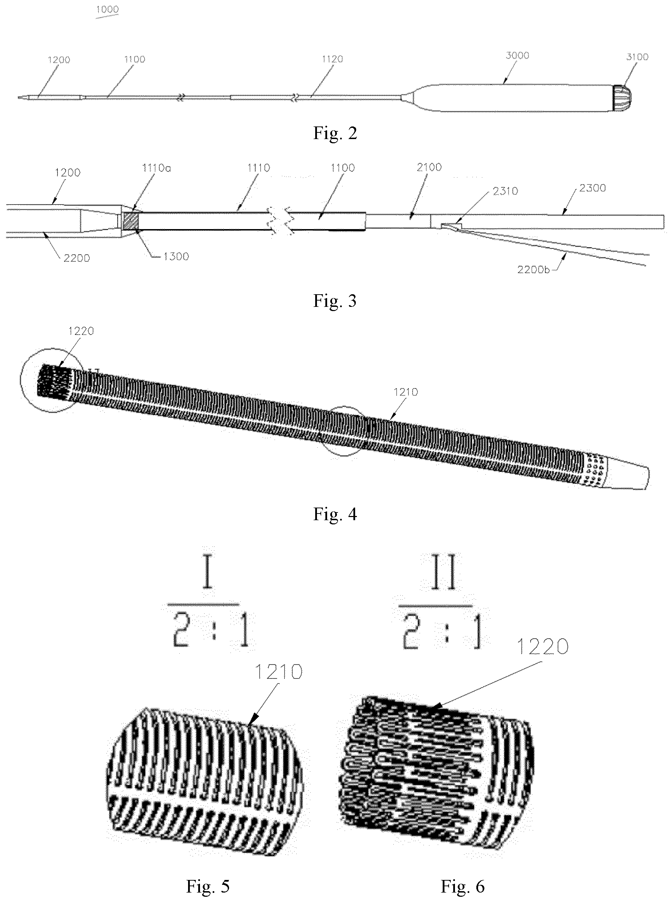

Referring to Figs. 4 to 6, in one embodiment, a reinforcement structure 1210 is provided over an outer surface of the bending outer catheter section 1200. The reinforcement structure 1210 may be provided in any suitable form. For example, the reinforcement structure 1210 may be reinforcement ribs provided on the outer surface of the bending outer catheter section 1200. The number of the reinforcement ribs may be two or more. The reinforcement ribs may be distributed along an axis of the bending outer catheter section 1200. Referring to Fig. 5, the reinforcement structure 1210 may be provided as arc-shaped elements arranged around the bending outer catheter section 1200, the plurality of reinforcement structures are arranged in parallel. The arc-shaped elements may be arranged in symmetry at opposite sides of the bending outer catheter section 1200. This axial distribution of the reinforcement structure 1210 imparts good compression resistance and excellent bendability in a plane perpendicular to the reinforcement structure 1210.

Referring to Fig. 6, a distal end of the bending outer catheter section 1200 has a deformable segment 1220, which is switchable between a straight cylindrical configuration and a tapered cylindrical configuration. The deformable segment 1220 may comprise a flared opening in the tapered cylindrical configuration, which can provide guidance to the stent body D during its retrieval, and may be restored to the rest configuration, i.e., the straight cylindrical configuration, after the retrieval is completed. The deformable segment 1220 may be provided in various forms. For example, as shown in Fig. 6, the deformable segment 1220 may be provided as multiple straight struts arranged circumferentially around the bending outer catheter section 1200. One ends of multiple straight struts are connected to the distal end of the bending outer catheter section 1200, forming a fixed diameter, while other ends of the plurality of straight struts are towards distal end and comprise a cantilevered arrangement. With this arrangement, when stressed radially inwards during retrieval of the stent body D, the straight struts will deflect inwardly, bringing the deformable segment 1220 into the tapered cylindrical configuration. Moreover, when not so stressed any more, it will restore the straight cylindrical configuration.

The proximal end of the bending outer catheter section 1200 has a diameter-varying segment joined to the distal end of the pushing outer catheter section 1100. The diameter of the diameter-varying segment of the bending outer catheter section 1200 gradually decreases from a distal end to a proximal end. The bending outer catheter section 1200 may match the bending inner catheter section 2200 in terms of length. For example, the diameter-varying segment of the proximal end of the bending inner catheter section 2200 may terminate at the diameter-varying segment of the proximal end of the bending outer catheter section 1200.

The pushing outer catheter section 1100 may be made up of layers of different materials. For example, the pushing outer catheter section 1100 may be a composite catheter made up of an outer polymer layer, an inner polymer layer, an outer-catheter braided layer and a distal metal ring. The outer polymer layer may be made of polyamide, a polyamide polyether copolymer or another material, and the inner polymer layer may be made of polytetrafluoroethylene, high-density polyethylene, polyamide, a polyamide-polyether copolymer or another material. The outer-catheter braided layer is located between the outer and inner polymer layers and may be braided with metal reinforcing wires. In order to reinforce the structure of the pushing outer catheter section 1100, the pushing outer catheter section 1100 may further arrange reinforcement structure 1210, such as reinforcement rib, in symmetry at opposite sides along the axis. Arranging the reinforcement ribs in symmetry at the opposite sides of the pushing outer catheter section 1100 can impart not only good bendability in a plane perpendicular to the reinforcement ribs but also desirable compression resistance.

In one embodiment, the distal end of the pushing outer catheter section 1100 may be joined to the proximal end of the bending outer catheter section 1200 by thermal fusion or the like. Additionally, a coupling ring 1300 may be provided between the distal end of the pushing outer catheter section 1100 and the proximal end of the bending outer catheter section 1200. The coupling ring 1300 may be sleeved over the joint location of the pushing outer catheter section 1100 and the bending outer catheter section 1200 and adjacent portion of the joint location, the coupling ring 1300 is used to enclose and reinforce the joint location between the distal end of the pushing outer catheter section 1100 and the proximal end of the bending outer catheter section 1200.

In order to reinforce structure of the pushing outer catheter section 1100, an outer-catheter reinforcement member 1110 may be provided on the pushing outer catheter section 1100, the outer-catheter reinforcement member 1110 may adopt reinforcement rib and the like, and the number of the reinforcement ribs may be two or more and the reinforcement ribs are arranged along an axis of the pushing outer catheter section 1100. The coupling ring 1300 may be fabricated using metal material, and the distal end of the pushing outer catheter section 1100 may be connected to the coupling ring 1300 by welding. Moreover, the coupling ring 1300 at the distal end of the pushing outer catheter section 1100 forms a welded coupling with the outer-catheter reinforcement member 1110, and a welded position 1110a is formed between the coupling ring 1300 and the outer-catheter reinforcement member 1110. This enables the outer-catheter reinforcement member 1110 to provide better support, ensuring stable release and retrieval of the stent body D and avoiding excessive stressing of the pushing inner catheter section 2100 or undesired dislodgement of the reinforcement ribs at the end portion, which may lead to a sharp drop in compression resistance, during retrieval of the stent body D.

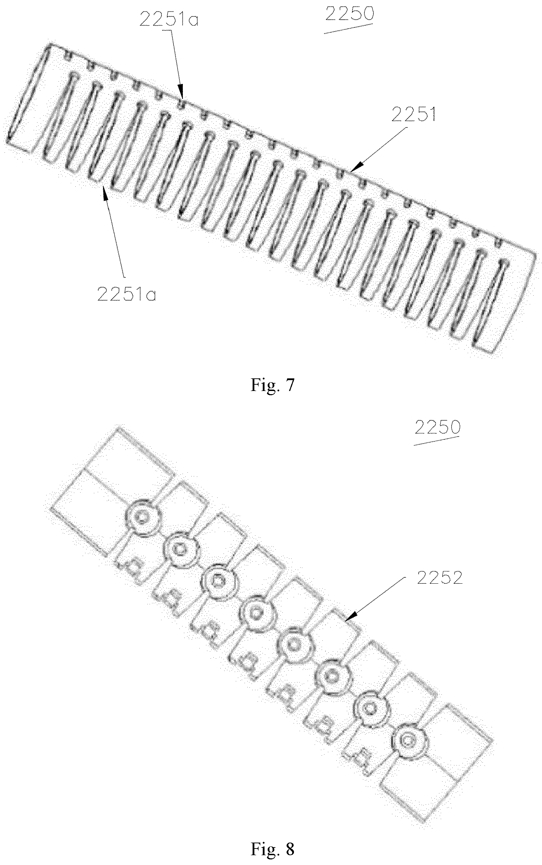

The bending inner catheter section 2200 includes a bendable backbone 2250 and a bendable protective layer. The bendable backbone 2250 is disposed within the bendable protective layer and serves as a backbone for the bending inner catheter section 2200, and the bendable protective layer wraps the backbone to provide protection to it. The bendable backbone 2250 may be provided in various forms. Referring to Fig. 7, in one embodiment, the bendable backbone 2250 includes a backbone tube 2251, and bend-assisting openings 2251a are formed in a wall of the backbone tube 2251. The bend-assisting opening 2251a is a linear opening and arranged circumferentially around the backbone tube 2251. The bend-assisting opening 2251a may surround at least a partial circumference of the backbone tube 2251. There are multiple bend-assisting openings 2251a. The multiple bend-assisting openings 2251a are arranged along an axis of the backbone tube 2251 and may be arranged in symmetry at opposite sides of the backbone tube 2251. The bend-assisting openings 2251a at one side may be staggered from those at the other side along the axis. Those skilled in the art may arrange the bend-assisting openings 2251a according to needs, without limiting the present application.

In one embodiment, the bendable protective layer may include an inner bendable protective layer and an outer bendable protective layer. The outer bendable protective layer is provided on an outer surface of the backbone tube 2251, and the inner bendable protective layer on an inner surface of the backbone tube 2251, to wrap the backbone tube 2251. The outer bendable protective layer may be made of a flexible polymer material such as polyurethane or a polyamide polyether copolymer, and the inner bendable protective layer may be made of a polymer such as polytetrafluoroethylene, high-density polyethylene, polyurethane or a polyamide polyether copolymer. The backbone tube 2251 may be made of a metal material, and the backbone tube 2251 with the bend-assisting openings 2251a may be fabricated by laser cutting.

Referring to Fig. 7, in one embodiment, the bendable backbone 2250 includes plural rotatable elements 2252 distributed along an axis of the bendable backbone 2250. Adjacent pivotable elements 2252 are articulated, and adjacent rotatable elements 2252 are rotationally coupled with fixed-axis, wherein the multiple rotation axes among the plurality of rotatable elements 2252 are mutually parallel. The rotatable elements 2252 may, for example, snake bone-like element, which are riveted together to form the backbone tube 2251. The riveted snake bone-like structure exhibits superior compliance, a force from the pull wire 2200b to be uniformly distributed across the backbone tube 2251, significantly lower the bending modulus of the backbone tube 2251, and the riveted snake bone-like structure exhibits good tensile performance. The bendable protective layer may be formed of a flexible polymer material such as polyurethane or a polyamide polyether copolymer, and may be directly arranged on the outer surface of the bendable backbone 2250.

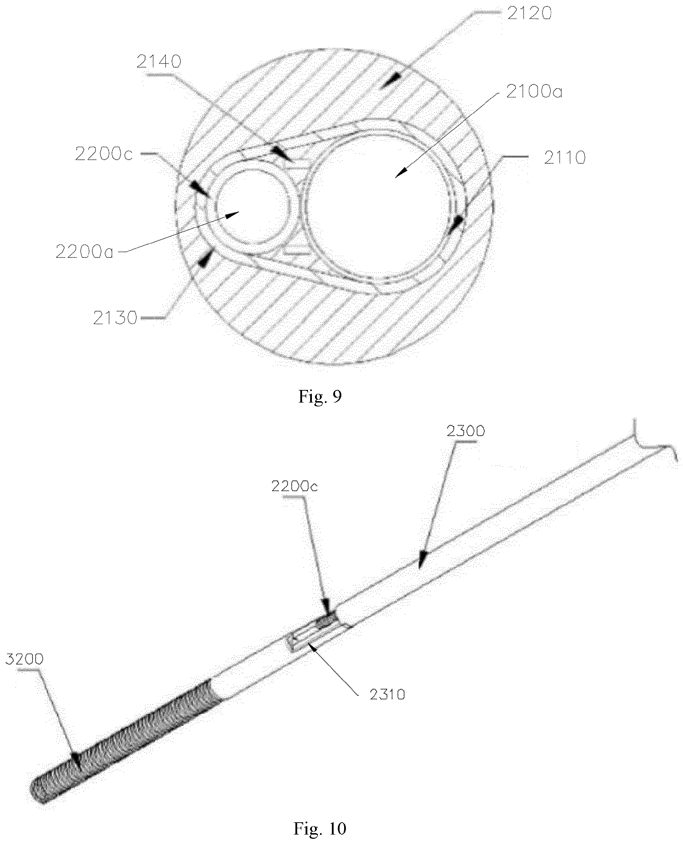

Referring to Fig. 9, in one embodiment, the pushing inner catheter section 2100 includes an inner-catheter inner layer 2110, an inner-catheter outer layer 2120 and an inner-catheter braided layer 2130 disposed between the inner-catheter inner and outer layers 2110 and 2120. At least one of the guide lumen 2100a and the pulling lumen 2200a is formed in the inner-catheter inner layer 2110. The inner-catheter outer layer 2120 may be made of a matrix material such as polyamide or a polyamide polyether copolymer, and the inner-catheter inner layer 2110 may be made of a self-lubricating polytetrafluoroethylene material, which imparts self-lubricating properties to the pulling lumen 2200a formed by the inner-catheter inner layer 2110. Inner-catheter reinforcement elements 2140 are provided within the inner-catheter inner layer 2110 located between the guide lumen 2100a and the pulling lumen 2200a.

A bend-resistant support tube 2200c is provided in the pulling lumen 2200a. The bend-resistant support tube 2200c exhibits bend resistance, and under the pulling force of the pull wire 2200b, the bend-resistant support tube 2200c enables the pushing inner catheter section 2100 that includes the pulling lumen 2200a to withstand greater stress and exhibit minimal deformation. As a non-limiting example, the bend-resistant support tube 2200c may be implemented as a dense compression spring tube or the like. The dense compression spring tube may be disposed between the inner-catheter inner layer 2110 and the inner-catheter braided layer 2130, and the inner-catheter braided layer 2130 can provide enhanced hoop constraint. When the pull wire 2200b is tensioned, the inner catheter 2000 tends to contract under the action of a force along its axis. However, as the dense compression spring tube can provide sufficient support, the inner catheter 2000 will overall undergo only minimal deformation. When the inner catheter 2000 is bent, a force will be generated perpendicular to the axis. However, since the restriction of the inner-catheter braided layer 2130 can impart higher hoop stiffness, the inner catheter 2000 can be avoided from deforming circumferentially.

Referring to Fig. 10, a proximal end of the pushing inner catheter section 2100 is provided with an inner-catheter coupling segment 2300, and the pushing outer catheter section 1100 may be provided with a corresponding outer-catheter coupling segment 1120 configured to be sleeved over the inner-catheter coupling segment 2300. The inner-catheter coupling segment 2300 is provided with a wire exit opening 2310 that is in communication with the pulling lumen 2200a, and a proximal end of the pull wire 2200b is guided out through the opening. A distal end of the inner-catheter coupling segment 2300 is joined to the pushing inner catheter section 2100, and a proximal end of the inner-catheter coupling segment 2300 is configured to be coupled to a grip member 3000 such as a handle. The inner-catheter coupling segment 2300 may be provided with a thread 3200 at its proximal end, and the inner-catheter coupling segment may be coupled to the grip member 3000 by threaded engagement. The grip member 3000 may be provided with a rotary control member 3100, which can be rotated to control tightening or loosening of the pull wire 2200b.The inner-catheter coupling segment 2300 may be a reinforcement tube made of metal material with structural reinforcement properties, and may consist of a first coupling segment 2300a and a second coupling segment 2300b. A proximal end of the first coupling segment 2300a is coupled to the grip member 3000 and a distal end of the first coupling segment 2300a is coupled to a proximal end of the second coupling segment 2300b. A distal end of the second coupling segment 2300b is coupled to the pushing inner catheter section 2100 by integral fusion. Such integral fusion can ensure smooth passage for overall bending control. In this case, the dense compression spring tube may extend from distal end to the wire exit opening 2310, ensuring unobstructed axial passage of the pull wire 2200b.

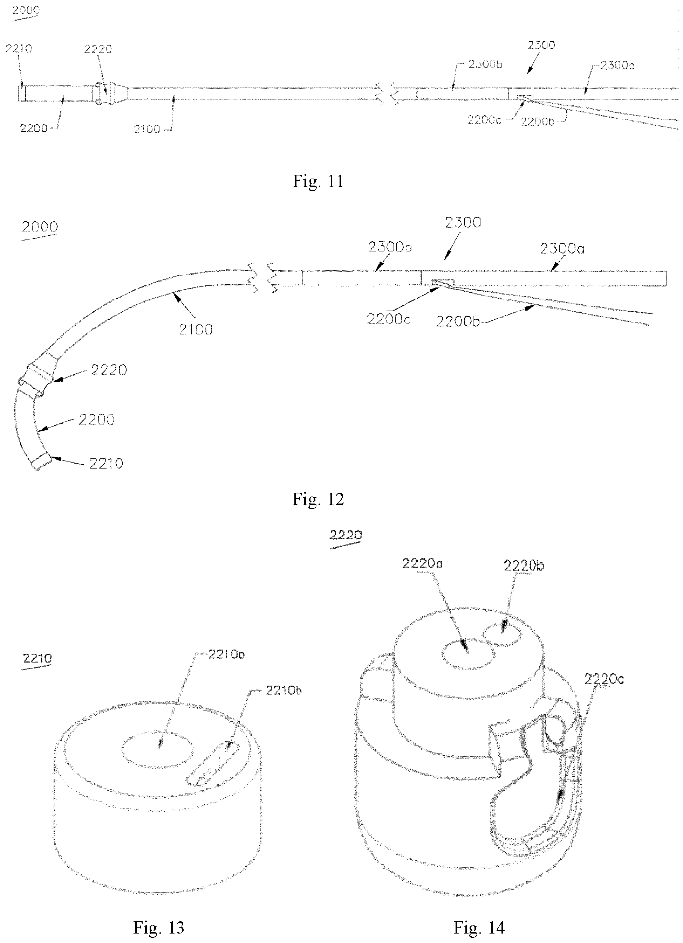

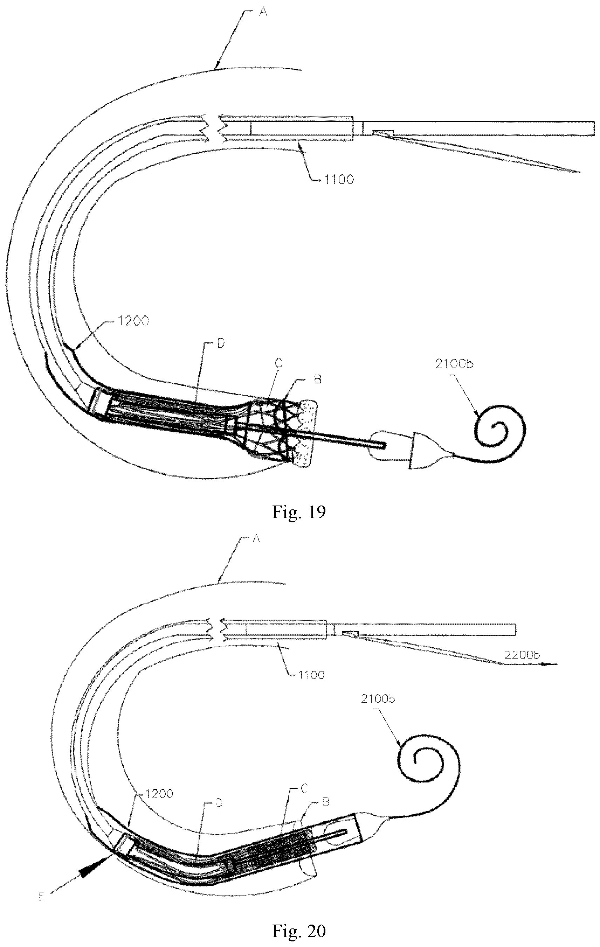

Referring to Figs. 11 to 21, the pulling fixing member 2210 is disposed at the distal end of the bending inner catheter section 2200. Referring to Fig. 13, the pulling fixing member 2210 comprises a first inner bore 2210a in communication with the guide lumen 2100a and allows the guide wire 2100b to exit from the distal end of the pulling fixing member 2210. The pulling fixing member 2210 is provided with a first wire attachment member 2210b, and the pull wire 2200b is connected to the first wire attachment member 2210b and hence connected to the distal end of the bending inner catheter section 2200.

The bending inner catheter section 2200 is provided at the proximal end thereof with a stent fixing member 2220, which couples the bending inner catheter section 2200 to the distal end of the pushing inner catheter section 2100. Referring to Fig. 14, the stent fixing member 2220 comprises a second inner bore 2220a in communication with the guide lumen 2100a. The second inner bore 2220a allows the guide wire 2100b to exit from the proximal end of the stent fixing member 2220. The stent fixing member 2220 has a stent fixing element 2220c for securing the stent body D with the valve C. Specifically, a proximal portion of the stent body D with the valve C is arranged on the bending inner catheter section 2200 by the stent fixing member 2220, allowing the stent body D to be crimped into the bending inner catheter section 2200. The stent fixing member 2220 comprises a lateral pull-wire hole 2220b in communication with the pulling lumen 2200a. The proximal end of the pull wire 2200b may extends proximally by inserting in the lateral pull-wire hole 2220b and finally guided out through the wire exit opening 2310 in the inner-catheter coupling segment 2300.

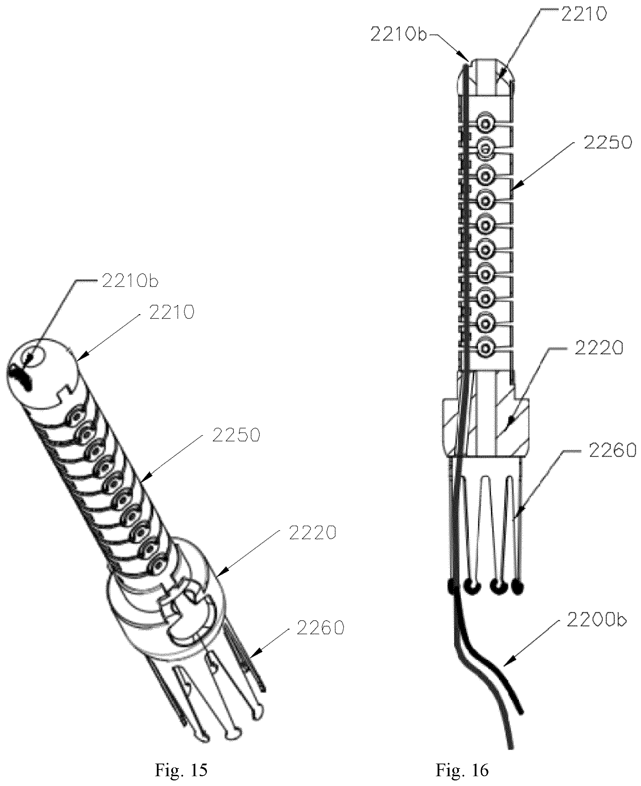

Referring to Figs. 15 and 16, the pulling fixing member 2210, the bendable backbone 2250, the stent fixing member 2220 and a fusion connection claw 2260 are welded into a fixed assembly. The pull wire 2200b can be connected to the pulling fixing member 2210 in multiple ways. For example, the pull wire 2200b may be suspended from the pulling fixing member 2210 by passing two pull wires 2200b proximally through the bendable backbone 2250 and then through the lateral pull-wire hole 2220b of the stent fixing member 2220. Next, the fusion connection claw 2260 is thermally bonded to and wraps the distal end of the pushing inner catheter section 2100. This configuration ensures that the lateral pull-wire hole 2220b of the stent fixing member 2220 communicates with the guide lumen 2200a of the inner catheter 2000, thereby establishing a complete pulling lumen 2200a.

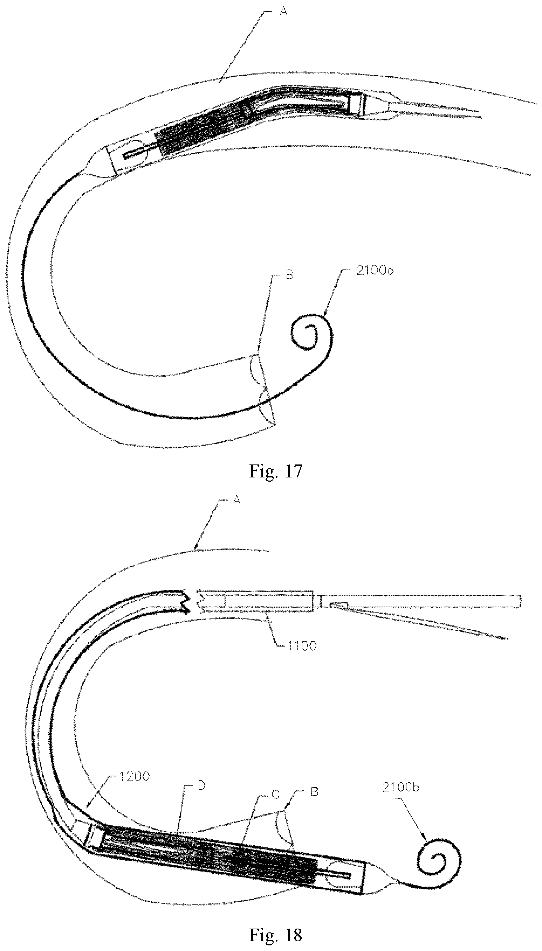

Referring to Figs. 17 to 21, when applying a pull force that is oriented to the proximal end to the pull wire 2200b, both the bending inner catheter section 2200 and the bending outer catheter section 1200 may be bent to a certain extent depending on their bending moduli and lengths. In order to ensure performance of the valve C, the lengths of the bending inner catheter section 2200 and the bending outer catheter section 1200 may be less than a distance between the pulling fixing member 2210 and the valve C.

After the stent body D with the valve C is loaded in the inner catheter 2000 and the outer catheter 1000 of the delivery system, the inner catheter 2000 and the outer catheter 1000 may be advanced through the aortic arch A. If resistance is encountered, apply small-angle deflection to both the bending inner catheter section 2200 and bending outer catheter section 1200 before advancing. After deflection, the distal ends of both the bending inner catheter section 2200 and bending outer catheter section 1200, the pull wire 2200b will disengage from the blood vessel wall, facilitating the advancement. If the inner catheter 2000 and the outer catheter 1000 were not steerable, then after being advanced through the aortic arch A and the valvular annulus, they would be overall straight and oriented at a relative large angle with respect to a plane of the annulus B, and if the stent body D were released at this time, it would possibly fall off or be tilted. In contrast, according to the present application, upon crossing the annulus, perform deflection adjustment of both the inner catheter 2000 and outer catheter 1000. the stent body D, the guide wire 2100b will bend with the inner catheter 2000 and outer catheter 1000, resulting in the inner catheter 2000 and the outer catheter 1000 being oriented normal to plane of the annulus B. The angle at which the inner catheter 2000 and the outer catheter 1000 are bent may depend on the actual anatomy of the arch, and the present application is not particularly limited in this regard.

The controlled deflection adjustment of both the inner catheter 2000 and outer catheter 1000 enables bending control of the stent body D. The adjustment of bending angles enables coaxial deployment suitable for over 98% of patient anatomies. In a procedure, the outer catheter 1000 may be retracted by manipulating on push button on the handle, and the inner catheter 2000 maintains stable bent throughout the entire releasing process via its controlled bending mechanism, and the stent body D remains perpendicular to the plane of the annulus B. This efficiently ensures stable and accurate release of the stent body D at a target site. Additionally, the stent body D is passively steered at a rear end, and after stable anchoring of the stent body D is attained, it is released from steering action for complete release. The anchoring of the stent body D is not affected at all.

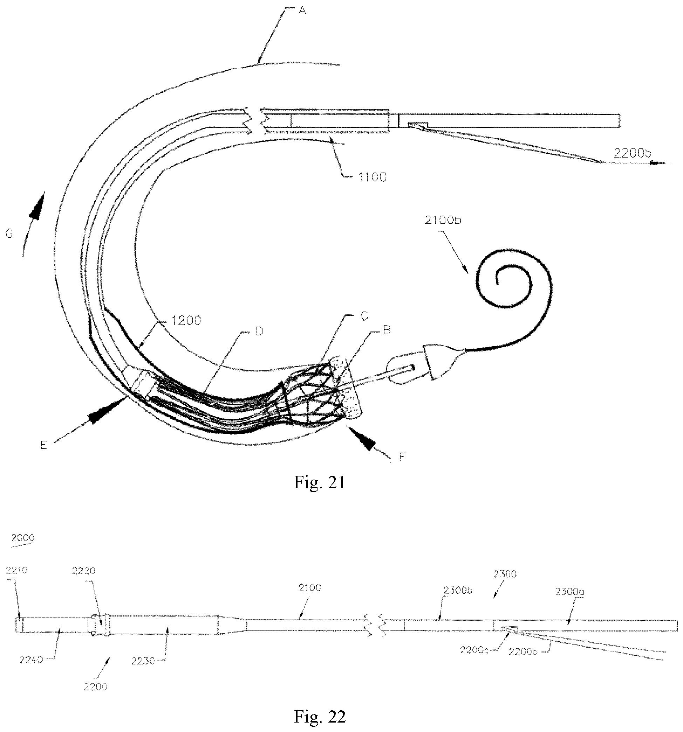

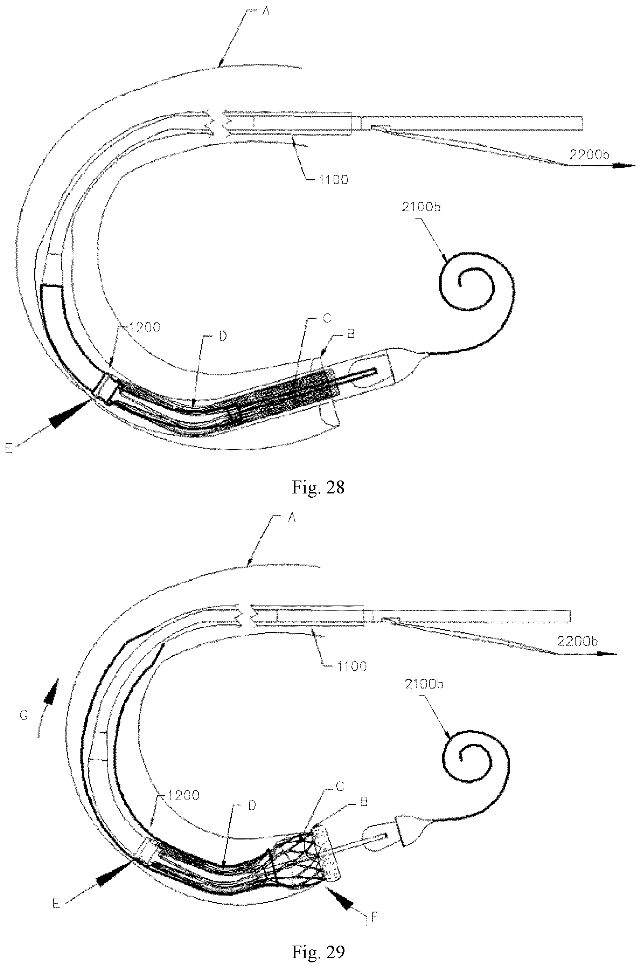

Referring to Figs. 22 to 29, the bending inner catheter section 2200 may include a first inner-catheter segment 2230 and a second inner-catheter segment 2240, along an axis. As non-limiting alternatives to this two-segment structure, the bending inner catheter section 2200 may also include three or more segments. A pulling fixing member 2210 may be provided at a distal end of the second inner-catheter segment 2240 and comprise a first inner bore 2210a. the first inner bore 2210a communicates with the guide lumen 2100a and is configured to permit exit of the guide wire 2100b from the distal end of the pulling fixing member2210. The pulling fixing member 2210 may have a first wire attachment member 2210b, and the pull wire 2200b may be connected to the first wire attachment member 2210b and hence connected to the distal end of the bending inner catheter section 2200.

A distal end of the first inner-catheter segment 2230 may be coupled to a proximal end of the second inner-catheter segment 2240 by a stent fixing member 2220 comprising a second inner bore 2220a, the second inner bore 2220a communicates with the guide lumen 2100a and is configured to permit exit of the guide wire 2100b from the proximal end of stent fixing member 2220. The stent fixing member 2220 may have a stent fixing element 2220c for securing the stent body D with the valve C. Specifically, a proximal portion of the stent body D with the valve C is sleeved on the bending inner catheter section 2200 by the stent fixing member 2220, allowing the stent body D to be crimped into the bending inner catheter section 2200. The stent fixing member 2220 may comprise a lateral pull-wire hole 2220b in communication with the pulling lumen 2200a. The proximal end of the pull wire 2200b may extend proximally by inserted in the lateral pull-wire hole 2220b and finally guided out through the wire exit opening 2310 in the inner-catheter coupling segment 2300.

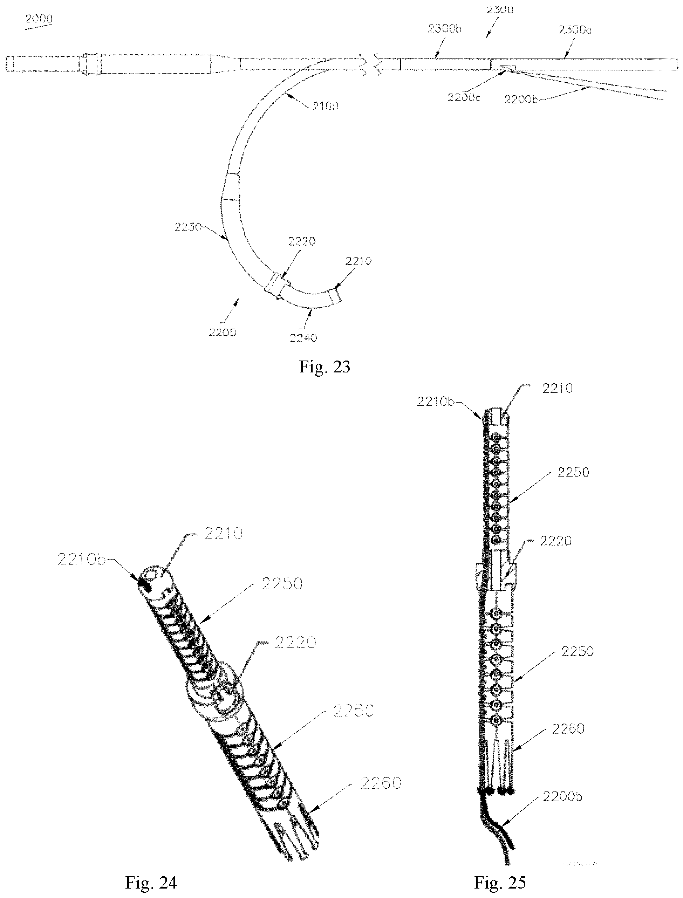

Referring to Figs. 24 and 25, the pulling fixing member 2210, the bendable backbone 2250 in the second inner-catheter segment 2240, the stent fixing member 2220 and the bendable backbone 2250 in the first inner-catheter segment 2230 and a fusion connection claw 2260 are welded into a fixed assembly. The pull wire 2200b may be connected to the pulling fixing member 2210 in various manners. For example, the pull wire 2200b may be suspended from the pulling fixing member 2210 by passing two pull wires 2200b proximally sequentially through the bendable backbone 2250 in the second inner-catheter segment 2240, the lateral pull-wire hole 2220b in the stent fixing member 2220 and the bendable backbone 2250 in the first inner-catheter segment 2230. The fusion connection claw 2260 is thermally bonded to and wraps the distal end of the pushing inner catheter section 2100. This configuration ensures that the lateral pull-wire hole 2220b of the stent fixing member 2220 communicates with the guide lumen 2200a of the inner catheter 2000, thereby establishing a complete pulling lumen 2200a.

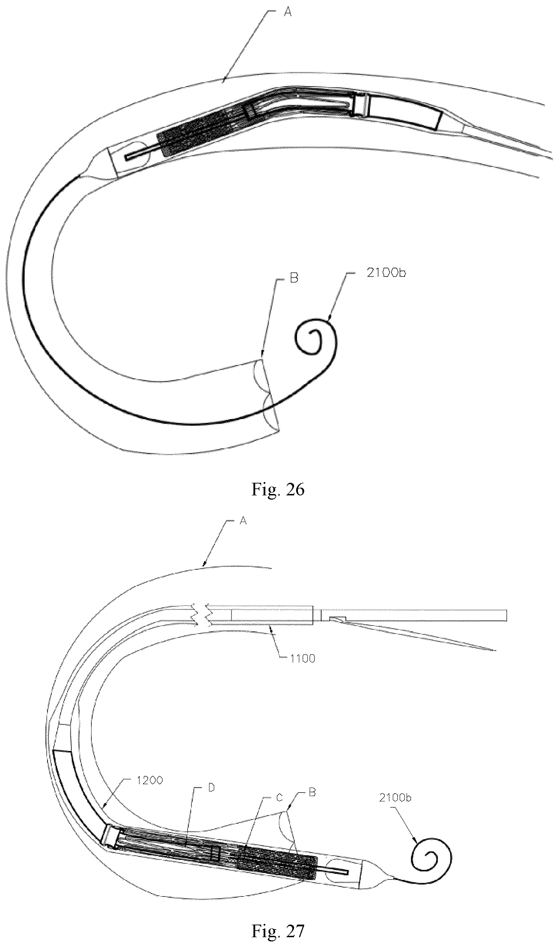

Referring to Figs. 26 to 29, when applying a pull force that is oriented to the proximal end to the pull wire 2200b, both the bending inner catheter section 2200 and the bending outer catheter section 1200 may be bent to a certain extent depending on their bending moduli and lengths. In order to ensure performance of the valve C, the lengths of the bending inner catheter section 2200 and the bending outer catheter section 1200 may be less than a distance between the pulling fixing member 2210 and the valve C. Since the bending inner catheter section 2200 is a two-segment structure including the first inner-catheter segment 2230 and the second inner-catheter segment 2240 along the axis, it can be bent at a large angle at a given force being applied thereto.

After the stent body D with the valve C is loaded in the inner catheter 2000 and the outer catheter 1000 of the delivery system, the inner catheter 2000 and the outer catheter 1000 may be advanced through the aortic arch A. If resistance is encountered, apply small-angle deflection to both the bending inner catheter section 2200 and bending outer catheter section 1200 before advancing. After deflection, the distal ends of both the bending inner catheter section 2200 and bending outer catheter section 1200, the pull wire 2200b will disengage from the blood vessel wall, facilitating the advancement. After crossing the valvular annulus, performing deflection adjustment of both the inner catheter 2000 and outer catheter 1000. The stent body D, the guide wire 2100b will bend with the inner catheter 2000 and outer catheter 1000, the inner catheter 2000 and the outer catheter 1000 will be oriented normal to plane of the annulus B. The angle at which the inner catheter 2000 and the outer catheter 1000 are bent may depend on the actual anatomy of the arch, and the present application is not particularly limited in this regard.

The two-segment structure of the bending inner catheter section 2200 that includes the first inner-catheter segment 2230 and the second inner-catheter segment 2240 along the axis entails an effort-saving design suitable for use in 90% of patients and in a wider range of applications. In a procedure, the outer catheter 1000 may be retracted by manipulating on push button on the handle, and the inner catheter 2000 maintains stable bent throughout the entire releasing process via its controlled bending mechanism, and the stent body D remains perpendicular to the plane of the annulus B. This efficiently ensures stable and accurate release of the stent body D at a target site. Additionally, the stent body D is passively steered at a rear end, and after stable anchoring of the stent body D is attained, it is released from steering action for complete release. Therefore, the anchoring of the stent body D is not affected at all.

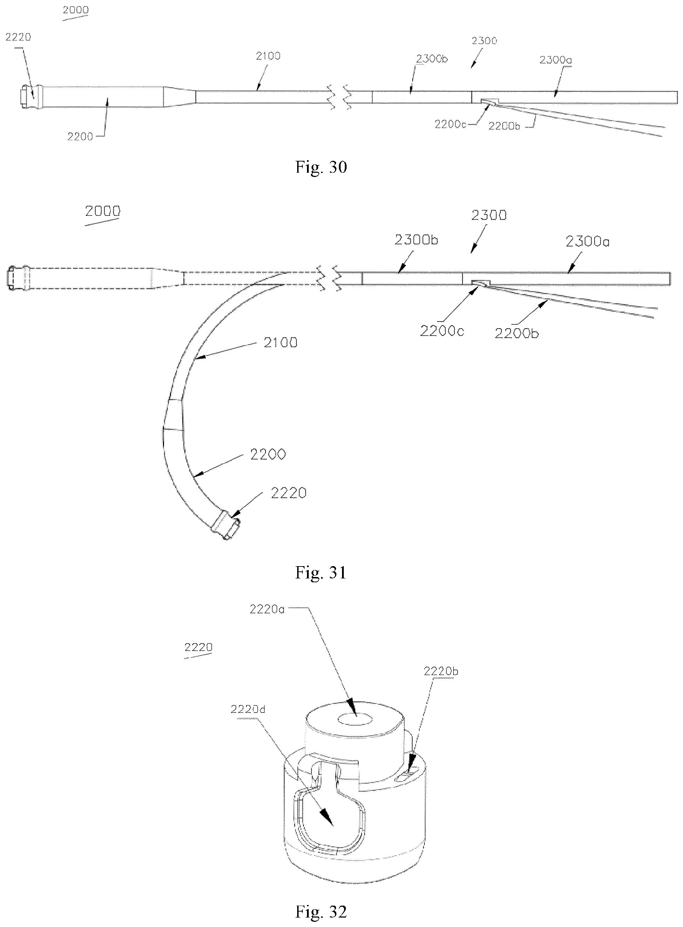

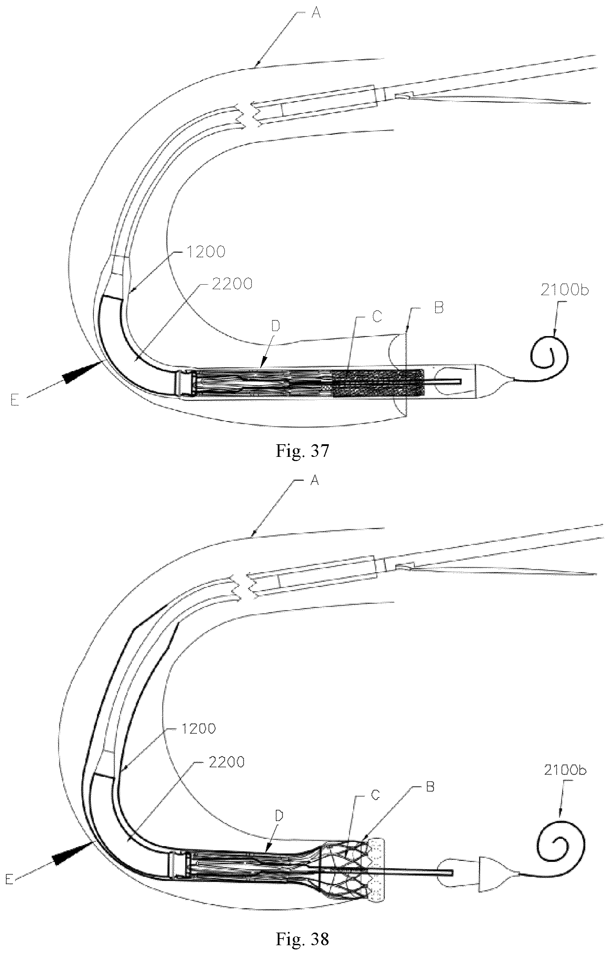

Referring to Figs. 30 to 38, the stent fixing member 2220 may be provided at the distal end of the bending inner catheter section 2200, and the proximal end of the bending inner catheter section 2200 may be directly joined to the pushing inner catheter section 2100. The stent fixing member 2220 may comprise a second inner bore 2220a, the second inner bore 2220a communicates with the guide lumen 2100a and is configured to permit exit of the guide wire 2100b from the distal end of the stent fixing member 2220. The stent fixing member 2220 may have a stent fixing element 2220c for securing the stent body D with the valve C, the stent body D with the valve C does not cover the bending inner catheter section 2200. The stent fixing member 2220 may further have a second wire attachment member 2220d, and the pull wire 2200b may be connected to the second wire attachment member 2220d. With this arrangement, the stent fixing member 2220 functions both to secure the stent body D and connect the pull wire 2200b at the distal end of the bending inner catheter section 2200.

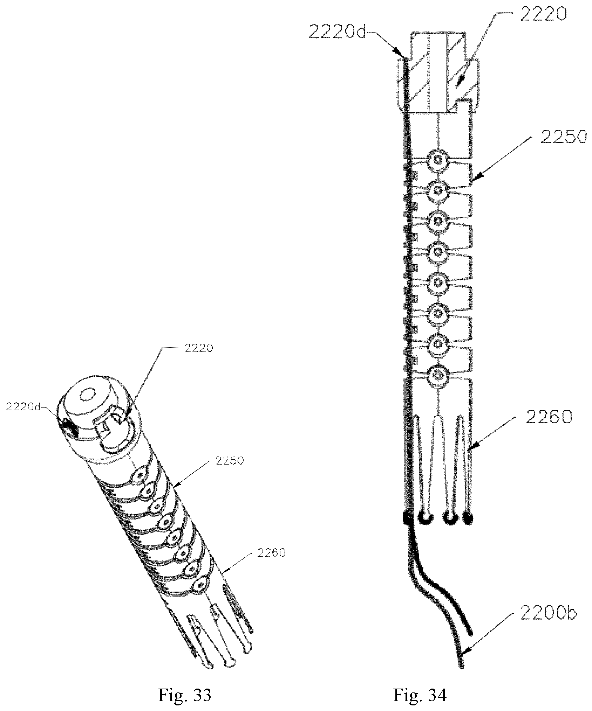

Referring to Figs. 33 and 34, the stent fixing member 2220 and the bendable backbone 2250 and fusion connection claw 2260 are welded into a fixed assembly. The pull wire 2200b may be connected to the stent fixing member 2220 in various manners. For example, the pull wire 2200b may be suspended from the pulling fixing member 2210 by passing two pull wires 2200b proximally through the bendable backbone 2250 and then pulling lumen 2200a of the inner catheter 2000. The fusion connection claw 2260 is thermally bonded to and wraps the distal end of the pushing inner catheter section 2100.

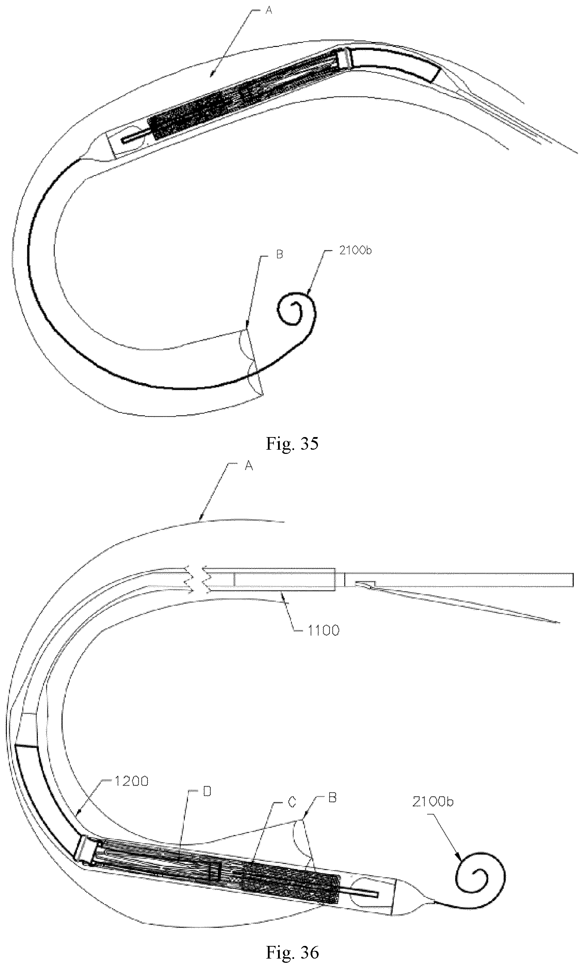

Referring to Figs. 35 to 38, when applying a pull force that is oriented to the proximal end to the pull wire 2200b, both the bending inner catheter section 2200 and the bending outer catheter section 1200 may be bent to a certain extent depending on their bending moduli and lengths. In order to ensure performance of the valve C, the lengths of the bending inner catheter section 2200 and the bending outer catheter section 1200 may be less than a distance between the pulling fixing member 2210 and the valve C.

In these embodiments, since the stent body D is secured on the stent fixing member 2220 at said distal end, it is not possible to bend the stent body D by steering the bending inner catheter section 2200 and the bending outer catheter section 1200. Accordingly, they have a relatively large straight length. This arrangement is suitable for use in patients with a long ascending aorta and can achieve coaxial release in over 80% of target patients. After the stent body D with the valve C is loaded in the inner catheter 2000 and the outer catheter 1000 of the delivery system, the inner catheter 2000 and the outer catheter 1000 may be advanced through the aortic arch A. If resistance is encountered, apply small-angle deflection to both the bending inner catheter section 2200 and bending outer catheter section 1200 before advancing. After deflection, the distal ends of both the bending inner catheter section 2200 and bending outer catheter section 1200, the pull wire 2200b will disengage from the blood vessel wall, facilitating the advancement, and enabling the bending inner catheter section 2200 and the bending outer catheter section 1200 to be perpendicular to the plane of the annulus B.

In a procedure, the outer catheter 1000 may be retracted by manipulating on push button on the handle, and the inner catheter 2000 maintains stable bent throughout the entire releasing process via its controlled bending mechanism, and the stent body D remains perpendicular to the plane of the annulus B. This efficiently ensures stable and accurate release of the stent body D at a target site. Additionally, the stent body D is passively steered at a rear end, and after stable anchoring of the stent body D is attained, it is released from steering action for complete release. Therefore, the anchoring of the stent body D is not affected at all.

The various technical features of the foregoing embodiments may be combined in any way. Although not all such combinations have been described above for the sake of brevity, any of them is considered to fall within the scope of this specification as long as there is no contradiction between the technical features.

Presented above are merely several embodiments of the present application. Although these embodiments are described with some particularity and in some detail, it should not be construed that they limit the scope of the present application in any sense. It is to be noted that various variations and modifications can be made by those of ordinary skill in the art without departing from the concept of the present application. Accordingly, it is intended that all such variations and modifications are embraced within the scope of this application as defined in the appended claims.

## Claims

1. A delivery system, comprising:
an outer catheter comprising an axial lumen;
an inner catheter comprising a guide lumen, wherein the inner catheter is movably disposed in the axial lumen of the outer catheter, wherein the inner catheter comprises an pushing inner catheter section and an bending inner catheter section along an axial direction, wherein a distal end of the pushing inner catheter section is joined to a proximal end of the bending inner catheter section, and wherein a diameter of the bending inner catheter section is greater than a diameter of the pushing inner catheter section;
a guide wire inserted in the guide lumen of the inner catheter; and
a pull wire connected to the bending inner catheter section.

2. The delivery system of claim 1, wherein a distal end of the pull wire is connected to a distal end of the bending inner catheter section.

3. The delivery system of claim 1, wherein a pulling fixing member is provided on the bending inner catheter section, and wherein the pull wire is connected to the bending inner catheter section through the pulling fixing member.

4. The delivery system of claim 3, wherein the pulling fixing member comprises a first inner bore that is in communication with the guide lumen.

5. The delivery system of claim 3, wherein the pulling fixing member is provided with a first wire attachment member, wherein the pull wire is connected to the first wire attachment member.

6. The delivery system of claim 1, wherein the inner catheter comprises a pulling lumen, wherein the pull wire is inserted within the pulling lumen of the inner catheter.

7. The delivery system of claim 6, wherein a bend-resistant support tube is provided in the pulling lumen.

8. The delivery system of claim 6, wherein a proximal end of the pushing inner catheter section is provided with an inner-catheter coupling segment, wherein a wire exit opening is provided on the inner-catheter coupling segment, and wherein the wire exit opening is in communication with the pulling lumen, and wherein a proximal end of the pull wire is guided out through the wire exit opening.

9. The delivery system of claim 1, wherein a proximal end of the bending inner catheter section has a diameter-varying segment that is joined to the distal end of the pushing inner catheter section, wherein a diameter of the diameter-varying segment of the bending inner catheter section gradually decreases from a distal end to a proximal end.

10. The delivery system of claim 1, wherein the bending inner catheter section is provided with a stent pulling fixing member.

11. The delivery system of claim 10, wherein the stent fixing member is provided at the proximal end of the bending inner catheter section, and wherein the bending inner catheter section is connected to the distal end of the pushing inner catheter section through the stent pulling fixing member.

12. The delivery system of claim 10, wherein the stent fixing member is provided at a distal end of the bending inner catheter section.

13. The delivery system of claim 10, wherein the bending inner catheter section comprises a first inner-catheter segment and a second inner-catheter segment along an axial direction, wherein a distal end of the first inner-catheter segment is coupled to a proximal end of the second inner-catheter segment by the stent pulling fixing member.

14. The delivery system of claim 10, wherein the stent fixing member comprises a second inner bore that is in communication with the guide lumen.

15. The delivery system of claim 10, wherein the stent fixing member has a stent fixing element.

16. The delivery system of claim **1,** wherein the bending inner catheter section comprises a bendable backbone and a bendable protective layer, and wherein the bendable backbone is disposed within the bendable protective layer.

17. The delivery system of claim 16, wherein the bendable backbone comprises a backbone tube, and wherein a wall of the backbone tube is provided with a bend-assisting opening.

18. The delivery system of claim 17, wherein the bend-assisting opening is a linear opening, wherein the bend-assisting opening is arranged circumferentially around the backbone tube, wherein a plurality of bend-assisting openings are provided, and wherein the plurality of bend-assisting openings are distributed along an axis of the backbone tube.

19. The delivery system of claim 17, wherein the bendable protective layer comprises an inner bendable protective layer and an outer bendable protective layer, wherein the outer bendable protective layer is provided on an outer surface of the backbone tube, and wherein the inner bendable protective layer is provided on an inner surface of the backbone tube.

20. The delivery system of claim 16, wherein the bendable backbone comprises a plurality of rotatable elements that are distributed along an axis of the bendable backbone, wherein adjacent rotatable elements are articulated.

21. The delivery system of claim 20, wherein adjacent rotatable elements are rotationally coupled with fixed-axis, and wherein a plurality of rotatable axes of the plurality of rotatable elements are parallel.

22. The delivery system of claim 1, wherein the pushing inner catheter section comprises an inner-catheter inner layer and an inner-catheter outer layer, wherein an inner-catheter braided layer is disposed between the inner-catheter inner layer and the inner-catheter outer layer, and wherein at least one of the guide lumen and the pulling lumen is formed within the inner-catheter inner layer.

23. The delivery system of claim 22, wherein at least one inner-catheter reinforcement element is disposed within the inner-catheter inner layer and is located between the guide lumen and the pulling lumen.

24. The delivery system of claim **1,** wherein the outer catheter comprises a pushing outer catheter section and a bending outer catheter section along an axis direction, and wherein a distal end of the pushing outer catheter section is joined to a proximal end of the bending outer catheter section.

25. The delivery system of claim 24, wherein a distal end of the bending outer catheter section has a deformable segment that comprises a straight cylindrical configuration and a tapered cylindrical configuration, wherein the deformable segment is configured to transition between the straight cylindrical configuration and the tapered cylindrical configuration.

26. The delivery system of claim 24, wherein a proximal end of the bending outer catheter section has a diameter-varying segment that is joined to the distal end of the pushing outer catheter section, wherein a diameter of the diameter-varying segment of the bending outer catheter section gradually decreases from a distal end to a proximal end.
